(19) Europäisches Patentamt
European Patent Office
Office européen des brevets

(11) Numéro de publication : **0 328 541 B1**

(12) **FASCICULE DE BREVET EUROPEEN**

(45) Date de publication du fascicule du brevet :
25.09.91 Bulletin 91/39

(51) Int. Cl.⁵ : **A61F 13/20**

(21) Numéro de dépôt : **87907230.4**

(22) Date de dépôt : **27.10.87**

(86) Numéro de dépôt international :
**PCT/FR87/00420**

(87) Numéro de publication internationale :
**WO 88/03398 19.05.88 Gazette 88/11**

(54) **MICRO-COMPRESSE OU PANSEMENT HEMORROIDAL ET SON PROCEDE DE FABRICATION.**

(30) Priorité : 03.11.86 FR 8615644

(43) Date de publication de la demande :
23.08.89 Bulletin 89/34

(45) Mention de la délivrance du brevet :
25.09.91 Bulletin 91/39

(84) Etats contractants désignés :
BE CH DE FR GB IT LI SE

(56) Documents cités :
FR-A- 1 488 600
FR-A-14 758 78
GB-A-83 710 6
GB-A-99 180 5
US-A-45 953 92

(73) Titulaire : BOISSERPE, Jean-Pierre
2, ruelle des Amoureux Garbejaire 107
F-06560 Valbonne (FR)

(72) Inventeur : BOISSERPE, Jean-Pierre
2, ruelle des Amoureux Garbejaire 107
F-06560 Valbonne (FR)

(74) Mandataire : Hautier, Jean-Louis
Cabinet Hautier Office Méditerranéen de
Brevets d'Invention et de Marques 24 rue
Masséna
F-06000 Nice (FR)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un pansement relevant du domaine médical, destiné à empêcher le boursoufflement externe des hémorroïdes (fluxion ou prolapsus hémorroïdal), afin de supprimer les douleurs qu'il provoque, ainsi qu'un procédé de fabrication de ce pansement.

Les soins actuellement dispensés, en dehors de l'intervention chirurgicale, se limitent à des applications de pommades et d'onguents, alliés à l'absorbtion de médicaments.

La majorité des patients redoute l'intervention chirurgicale et l'application de pommades réduit mais ne supprime pas les maux.

FR-A-1488600 décrit un pansement hémorroïdal constitué d'une couche de gaze enroulée sur elle-même avant d'être engagé entre les fesses. Ce pansement est inapproprié pour une introduction dans l'anus, et n'empêche donc pas de façon efficace la fluxion hémorroïdale

Le pansement selon l'invention permet à l'utilisateur de vivre sa vie quotidienne sans souffrance et sans crainte d'une crise en circonstances inopportunes : lieu de travail, lieu public etc...

La formation du boursoufflement hémorroïdal ou fluxion est empêchée par le pansement selon l'invention, objet de la revendication 1, qui interdit ainsi douleur, gêne et saignements.

Il est constitué à partir de deux éléments : sur une face d'une feuille en fibres de papier ou plutôt de ouate de cellulose très fine, de l'ordre de 20 grammes/m² environ, sur l'autre face d'une couche de gaze tissée en fil de lin ou de coton.

Ces éléments sont assemblés par thermocollage ou par tout autre procédé donnant le même résultat.

La surface approximative du pansement doit être de 20 cm², 4.5 cm × 4.5 cm pour une forme carrée, de 5 cm de ∅ pour une forme ronde.

La face papier ou ouate de cellulose peut servir de support à une poudre hémostatique (non indispensable).

La mise en place du pansement implique préalablement de rentrer la ou les hémorroïdes, d'assècher les parois anales et le sphincter, et pour faciliter l'adhérence de ne pas appliquer de corps gras (pommades etc...).

Placer le pansement face gaze sur le doigt, face papier ou ouate de cellulose supérieure puis l'introduire dans l'anus jusqu'au sphincter (profondeur de 3 à 4 cm).

Après application, la face papier ou ouate de cellulose du pansement adhère aux parois anales par absorption des particules humides, comme une feuille de papier à cigarette sur une coupure de rasoir. La face gaze, qui sert de support à la face papier, se retrouve par sa nouvelle position cônique, après retrait du doigt, gaze sur gaze et sa particularité rugueuse retient le pansement en place en empêchant le glissement vers l'extérieur.

La pression naturelle du sphincter retient le pansement sans problème 24 heures, même en situation debout prolongée et quelle que soit l'activité professionnelle exercée. La très faible épaisseur du pansement, de l'ordre de 2 à 3/10 de millimètre n'entraîne aucune gêne.

Le pansement peut être commercialisé en sachets individuels stérilisés.

Les procédés objets des revendications 5 et 6 sont particulièrement adaptés à la fabrication du pansement selon la revendication 1.

Il est donné à titre d'exemple non limitatif un mode de réalisation se référant aux dessins ci-annexés :

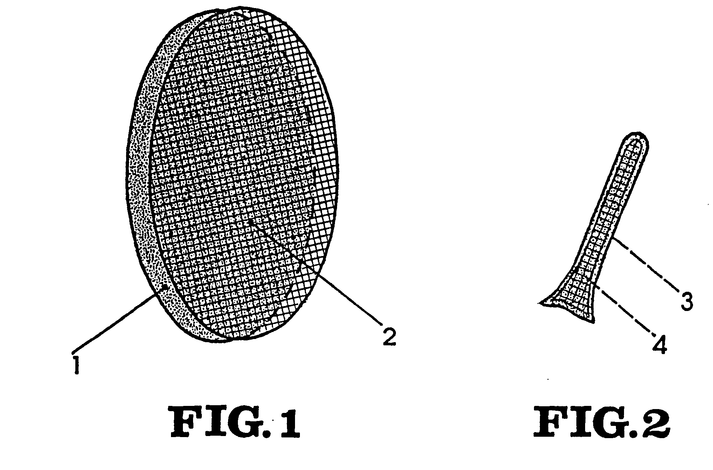

Figure 1 : vue en coupe décomposée et de profil du pansement.

Figure 2 : vue du pansement en position fonctionnelle.

D'une surface de 20 cm² environ, il est constitué sur une face d'une feuille de fibres de papier ou ouate de cellulose (1) doublée sur l'autre face d'une couche de gaze tissée en fil de lin ou de coton (2).

Le procédé de fabrication par thermocollage, préféré dans le cas présent au collage traditionnel, présente des particularités intéressantes de perméabilité à l'air et à l'eau et de ne pas se décoller même en cas de stérilisation à la vapeur.

Le procédé selon l'invention, papier ou ouate de cellulose thermocollé sur du tissu, représente une nouveauté et est susceptible d'être retenu par les industriels, notamment du secteur textile.

Il est à noter également que les dimensions dont fait état la présente description sont données à titre indicatif et exclusivement liées à la fabrication de la microcompresse dans son utilisation de pansement hémorroïdal. Il va de soi, que le même produit utilisé dans des buts différents pourra varier dans ses dimensions, tant dans le domaine médical ou paramédical que celui du secteur industriel notamment textile ou tout autre domaine.

## Revendications

1. Pansement hémorroïdal destiné à empêcher la fluxion hémorroïdale comprenant une couche de gaze (2) tissée en fil de lin ou de coton caractérisé en ce qu'il comprend sur une face de la couche de gaze une feuille (1) très fine de ouate de cellulose ou de fibres de papier, l'épaisseur de la couche de gaze et de la feuille et la dimension du pansement étant telles que l'application à sec et sans corps gras de la microcompresse en position cônique, face ouate de cellulose supérieure pour être en contact direct avec la peau des parois anales (3) permette, par la particularité de la cellulose, l'adhérence à la peau par absorption des particules humides, la face gaze repliée sur elle même gaze sur gaze (4) retenant par sa rugosité naturelle la micro-compresse en position interne permanente sous la pression naturelle du sphincter.

2. Pansement selon la revendication 1 caractérisé en ce qu'il présente une épaisseur très faible pour empêcher toute gêne au patient, de l'ordre de 0,2 à 0,3 mm.

3. Pansement selon la revendication 1 caractérisé en ce qu'il présente une surface de 20 cm² environ, surface nécessaire pour l'adhérence interne et suffisante pour éviter une gêne externe au patient et que sa forme peut être ronde.

4. Pansement selon la revendication 1 caractérisé en ce que la feuille de ouate de cellulose ou de fibres de papier supporte une poudre hémostatique.

5. Procédé de fabrication d'un pansement selon l'une quelconque des revendications 1 à 4 dans lequel une feuille (1) très fine de ouate de cellulose ou de fibres de papier est disposée sur une face d'une couche de gaze (2) tissée en fil de lin ou de coton, puis assemblée par thermocollage avec ladite couche de gaze (2).

6. Procédé de fabrication du pansement selon les revendications 1 et 2 utilisable également pour usages médicaux, paramédicaux ou industriels caractérisé par le thermocollage d'une feuille de ouate de cellulose sur la surface d'un tissu gaze.

## Patentansprüche

1. Hämorrhoidal-Verband, der zur Verhinderung einer Hämorrhoidalentzündung bestimmt ist und eine aus Leinen- oder Baumwollgewebe gewebte Gaze-Schicht (2) umfaßt, dadurch gekennzeichnet, daß er auf einer Seite der Gaze-Schicht eine sehr feine Folie (1) aus Zellulosewatte oder Papierfasern aufweist, wobei die Dicke der Gaze-Schicht und der Folie und die Abmessungen des Verbandes derart sind, daß die trockene, fettkörperfreie Anlegung der Mikrokompresse in konischer Position, in der sich die Zellulosewatte-Seite oben befindet, um in direkten Kontakt mit der Haut der Analwände (3) zu gelangen, durch die Besonderheit der Zellulose das Anhaften an der Haut durch die Absorption von Feuchtigkeitspartikeln ermöglicht, wobei die Gaze für Gaze übereinander gefaltete Gazeseite (4) durch ihre natürliche Rauhheit die Mikrokompresse unter dem natürlichen Druck des Schließmuskels in einer dauerhaften inneren Position hält.

2. Verband gemäß Anspruch 1, dadurch gekennzeichnet, daß er zur Vermeidung jeglicher Unannehmlichkeit für den Patienten eine sehr geringe Dicke in der Größenordnung zwischen 0,2 und 0,3 mm besitzt.

3. Verband gemäß Anspruch 1, dadurch gekennzeichnet, daß er eine Oberfläche von ungefähr 20 cm² besitzt, wobei diese Fläche zum inneren Anhaften notwendig ist und zur Vermeidung einer externen Unannehmlichkeit für den Patienten ausreicht, und daß seine Form rund sein kann.

4. Verband gemäß Anspruch 1, dadurch gekennzeichnet, daß auf der Folie aus Zellulosewatte oder aus Papierfasern ein blutstillendes Puder aufgebracht ist.

5. Verfahren zur Herstellung eines Verbandes gemäß einem der Ansprüche 1 bis 4, in dem eine sehr feine Folie (1) aus Zellulosewatte oder aus Papierfasern auf einer Seite einer Gaze-Schicht (2), die aus Leinen- oder Baumwollgarn gewebt ist, angeordnet wird und anschließend mittels Thermoklebung mit der Gaze-Schicht (2) zusammengefügt wird.

6. Verfahren zur Herstellung des Verbandes gemäß den Ansprüchen 1 und 2, der gleichermaßen für medizinische, paramedizinische oder industrielle Zwecke verwendbar ist, gekennzeichnet durch die Thermoklebung einer Folie aus Zellulosewatte auf die Oberfläche eines Gaze-Gewebes.

## Claims

1. Hemorrhoidal dressing adapted to prevent hemorrhoidal inflammation comprising a layer of gauze (2) woven of linen or cotton threads characterized in that it comprises on one surface of the layer of gauze a very thin sheet (1) of cellulose padding or paper fibers, the thickness of the layer of gauze and of the sheet and the size of the dressing being such that the dry application without greasy materials of the micro-compress in a conical configuration, with the cellulose padding surface upward to be in direct contact with the skin of the anal walls (3) permits, by the characteristics of the cellulose, the adhesion to the skin by absorption of moist particles, the gauze surface bent down on itself gauze-on-gauze (4) retaining by its natural roughness the micro-compress in permanent internal position under the natural pressure of the sphincter.

2. Dressing according to claim 1 characterized in that it has a very small thickness to prevent any discomfort to the patient, of the order of 0.2 to 0.3 mm.

3. Dressing according to claim 1 characterized in that it has an area of about 20 cm², which is necessary for the internal adherence and sufficient to avoid external difficulty to the patient and that its shape can be round.

4. Dressing according to claim 1 characterized in that the sheet of cellulose padding or paper fibers supports a hemostatic powder.

5. Process for the production of a dressing according to any one of claims 1 to 4 in which a very thin sheet (1) of cellulose padding or of paper fibers is disposed on a surface of a layer of gauze (2) woven from linen or cotton threads, then assembled by thermobonding with said layer of gauze (2).

6. Process for the fabrication of the dressing according to claims 1 and 2 useful also for medical, paramedical or industrial purposes characterized by the thermobonding of a sheet of cellulose padding to the surface of a woven fabric.

# FIG. 1

# FIG. 2